# EUROPEAN PATENT APPLICATION

(11) **EP 4 610 360 A1**
(43) Date of publication of application: **03.09.2025**
(21) Application number: 23903753.4
(22) Date of filing: 02.11.2023
(51) Int. Cl.: C12N 15/81, C12N 9/90, C07K 14/79, C07K 14/76, C07K 14/62, C12R 1/84, C12R 1/885

(54) **METHOD FOR PRODUCING RECOMBINANT PROTEIN BY USING PDI DERIVED FROM TRICHODERMA SP. STRAIN**

(30) Priority: 15.12.2022 KR 20220175628
(71) Applicant: CJ CheilJedang Corporation, Seoul 04560 (KR)
(72) Inventor: SONG, Gyuhyeon, Seoul 04560 (KR); BAE, Hyun Won, Seoul 04560 (KR); BYUN, Hyo Jeong, Seoul 04560 (KR); PARK, Jinsub, Seoul 04560 (KR); KIM, Byeong Soo, Seoul 04560 (KR)
(74) Representative: Meissner Bolte Partnerschaft mbB
(86) International application number: PCT/KR2023/017380
(87) International publication number: WO 2024/128550

(57) **Abstract**

The present invention relates to a method for producing a recombinant protein by using a PDI derived from Trichoderma sp. strain. A variant yeast comprising a gene that encodes a PDI derived from Trichoderma sp. (the genus Trichoderma) strain, of the present invention, highly expresses a target protein, and thus is used to produce a target recombinant protein at a high yield.

## Description

### [TECHNICAL FIELD]

### Cross-reference to related application(s)

The present application claims the benefit of the priority based on Korean patent application No. 10-2022-0175628 filed on December 15, 2022, and the entire contents disclosed in the documents of the corresponding Korean patent application are incorporated as a part of the present description.

The present invention relates to a method for producing a recombinant protein using PDI derived from *Trichoderma* sp. strain.

### [BACKGROUND ART]

Host cells mainly used for producing a gene recombinant protein include *E. coli,* yeast (*S. cerevisiae*), and animal cells, CHO cells, and the like, and among them, the yeast has several advantages compared to *E. coli.* Yeast is a eukaryotic cell genetically identical to higher organisms different from *E. coli,* and has a similar transcription and translation system to genes derived from higher organisms, and can remove introns through splicing, and has a secretory organ similar to the Golgi apparatus of higher organisms, and therefore, it can produce and secret protein activated through post-translational modification. In addition, yeast has advantages that can secret and produce a gene recombinant protein more efficiently outside the cells compared to *E. coli,* to help separation and purification of this protein, and does not have a problem due to endotoxins, which should be removed in case of pharmaceutical protein, when a protein is produced in the yeast. *S. cerevisiae,* which is representatively used among these yeasts, has been actively researched by many researchers since production of a gene recombinant protein using it has been reported in 1981. However, in order to obtain a high expression rate in *S. cerevisiae,* a multi-copy plasmid is required, and when high-concentration cell culture is conducted in a large-scale fermenter, the distribution, copy number, stability and the like of these plasmids often become problems, and most of glycolytic gene-derived promoters are constitutive promoters, and thus, when protein is expressed using them, there is a problem that cells that have lost plasmids may be dominating, and there is a problem that promoters which can be controlled are also difficult to be accurately controlled. As an alternative to solve such problems, a facultative methylotrophic yeast, *Pichia pastoris* has been recently spotlighted. *Pichia pastoris* is a methylotrophic yeast, and a foreign gene may be integrated into chromosomal DNA, and as a promoter for enzymes on the pathway using methanol, AOX1 is used, and there is an advantage that this promoter is very strong.

Protein disulfide isomerase (PDI) is an enzyme that catalyze a thiol:disulfide bond exchange reaction, and stays in the endoplasmic reticulum and is newly synthesized and transfers a disulfide bond to a secretory protein entering the endoplasmic reticulum. PDI is oxidoreductase that has not only activity to transfer a disulfide bond, but also isomerization activity to break an incorrectly linked disulfide bond and convert it into a structurally stable disulfide bond. When the PDI does not function normally, substrates are degraded rather than departing from the endoplasmic reticulum and being secreted into the Golgi body or outside the cell. Therefore, depending on the degree of the activity of PDI, the efficiency of synthesis of secretory protein may vary.

Accordingly, there have been attempts to use PDI to produce a recombinant protein. Fusing thermophilic fungal PDI to the amino-terminus of a target protein and secreting it outside the cell in a *Bacillus brevis* strain (Kajino, T. et al. (2000) Appl. Environ. Microbiol. 66: 638-642), or coexpressing dsbC, one kind of PDI, with a target protein in oxidative cytoplasm of mutant *E. coli* (Bessette, P.H. et al. (1999) Proc. Natl. Acad. Sci. USA, 96: 13703-13708), and the like have been reported. However, it is known that when secreted outside the cell from a *Bacillus* train, there is a disadvantage of significantly low yield of the target protein due to problems such as secretion of protease that the *Bacillus* strain has, and the like, and when dsbC and the target protein are coexpressed in the cytoplasm, the expression rate is low.

### [DISCLOSURE]

### [TECHNICAL PROBLEM]

One embodiment of the present application
provides a method for producing a recombinant protein comprising;
1) preparing a mutant yeast comprising performing the following i) and ii) steps simultaneously, sequentially, or in reverse order:
   i) introducing a vector comprising a gene encoding PDI (protein disulfide isomerase) derived from *Trichoderma* sp. strain into a yeast;
   ii) introducing a vector comprising a gene encoding a target protein into a yeast; and
2) culturing the mutant yeast prepared in the step 1).

Another embodiment of the present application provides a mutant yeast for producing a target protein comprising a gene encoding PDI (protein disulfide isomerase) derived from *Trichoderma* sp. strain.

Other embodiment of the present application provides a composition for producing a target protein in a mutant yeast, comprising a gene encoding PDI (protein disulfide isomerase) derived from *Trichoderma* sp. strain or a vector comprising the same.

### [TECHNICAL SOLUTION]

In order to achieve the above purposes, the present invention provides a mutant yeast strain transformed with a gene encoding PDI (protein disulfide isomerase) derived from *Trichoderma* sp. strain and a method for producing a target protein using the same.

Hereinafter, the present invention will be described in more detail.

The present invention
provides a method for producing a recombinant protein comprising;
1) preparing a mutant yeast comprising performing the following i) and ii) steps simultaneously, sequentially, or in reverse order:
   i) introducing a vector comprising a gene encoding PDI (protein disulfide isomerase) derived from *Trichoderma* sp. strain into a yeast;
   ii) introducing a vector comprising a gene encoding a target protein into a yeast; and
2) culturing the mutant yeast prepared in the step 1).

The term used in the present description, "PDI (protein disulfide isomerase)" refers to an enzyme that catalyze a thiol:disulfide bond exchange reaction, and may be interchangeably used with "protein disulfide isomerase".

The PDI of step i) of step 1) above may be PDI derived from *Trichoderma* sp. strain, and preferably, may be PDI derived from *Trichoderma reesei* strain.

In one embodiment of the present invention, the PDI may comprise the amino acid sequence of SEQ ID NO: 14, or consist of the amino acid sequence of SEQ ID NO: 14, and a polypeptide having an amino acid sequence in which a part of the sequence of the amino acid sequence of SEQ ID NO: 14 is deleted, modified, substituted or added may be also included in the scope of the present application, as long as it has enzymatic activity identical or corresponding to PDI. In addition, regardless of the origin of the microorganism, a polypeptide having conversion activity identical or corresponding to PDI, which is a polypeptide having homology or identity of at least 60% or more, 70% or more, 80% or more, 85% or more, 90% or more, 92% or more, 94% or more, 96% or more, 98% or more, or 99% or more, 99.5% or more or 99.8% or more to the amino acid sequence of SEQ ID NO: 14, may be also included in the scope of the present application as the PDI.

The yeast of the step i) and step ii) of the step 1) above may be any one selected from the group consisting of genus *Pichia,* genus *Candida* and genus *Hansenula* strains. In one embodiment, the yeast may be *Pichia pastoris,* and more specifically, it may be *Pichia pastoris* BG16 strain, but not limited thereto.

The target protein of the step ii) of the step 1) may be a protein comprising a disulfide bond in the protein, and it may be a protein comprising at least 3 disulfide bonds in the protein, but not limited thereto.

In one embodiment of the present invention, the target protein may be any one or more selected from the group consisting of transferrin, albumin and insulin precursors, and the transferrin, albumin and insulin precursors may be bovine-derived proteins, but not limited thereto.

In the present description, the term, "transferrin" may man serum transferrin, and it may be used as one of factors replacing serum (FBS, fetal bovine serum) in serum free media (FBS free media) and the like. In one embodiment, the transferrin may comprise the amino acid sequence of SEQ ID NO: 6 below, or consist of the amino acid sequence of SEQ ID NO: 6, but not limited thereto.

[SEQ ID NO: 6] Bovine Transferrin (N495Q is indicated in bold)
DPERTVRWCTISTHEANKCASFRENVLRILESGPFVSCVKKTSHMDCIKAISN
NEADAVTLDGGLVYEAGLKPNNLKPVVAEFHGTKDNPQTHYYAVAVVKKDTDFKL
NELRGKKSCHTGLGRSAGWNIPMGKLYKELPDPQESIQRAAANFFSASCVPCADQSS

FPKLCQLCAGKGTDKCACSNHEPYFGYSGAFKCLMEGAGDVAFVKHSTVFDNLPNP
EDRKNYELLCGDNTRKSVDDYQECYLAMVPSHAVVARTVGGKEDVIWELLNHAQE
HFGKDKPDNFQLFQSPHGKDLLFKDSADGFLKIPSKMDFELYLGYEYVTALQNLRES
KPPDSSKDECMVKWCAIGHQERTKCDRWSGFSGGAIECETAENTEECIAKIMKGEAD
AMSLDGGYLYIAGKCGLVPVLAENYKTEGESCKNTPEKGYLAVAVVKTSDANINW
NNLKDKKSCHTAVDRTAGWNIPMGLLYSKINNCKFDEFFSAGCAPGSPRQSSLCALC
IGSEKGTGKECVPNSNERYYGYTGAFRCLVEKGDVAFVKDQTVIQNTDGNNNEAW
AKNLKKENFEVLCKDGTRKPVTDAENCHLARGPNHAVVSRKDKATCVEKILNKQQ
DDFGKSVTDCTSNFCLFQSNSKDLLFRDDTKCLASIAKKTYDSYLGDDYVRAMTNL
RQCSTSKLLEACTFHKP

In the present invention, the term, "albumin" may mean serum album, and it may be used as one of factors replacing serum in serum free media and the like. In one embodiment, the albumin may comprise the amino acid sequence of SEQ ID NO: 24 below, or consist of the amino acid sequence of SEQ ID NO: 24, but not limited thereto.

[SEQ ID NO: 24]
DTHKSEIAHRFKDLGEEHFKGLVLIAFSQYLQQCPFDEHVKLVNELTEFAKT
CVADESHAGCEKSLHTLFGDELCKVASLRETYGDMADCCEKQEPERNECFLSHKDD
SPDLPKLKPDPNTLCDEFKADEKKFWGKYLYEIARRHPYFYAPELLYYANKYNGVF
QECCQAEDKGACLLPKIETMREKVLASSARQRLRCASIQKFGERALKAWSVARLSQ
KFPKAEFVEVTKLVTDLTKVHKECCHGDLLECADDRADLAKYICDNQDTISSKLKEC
CDKPLLEKSHCIAEVEKDAIPENLPPLTADFAEDKDVCKNYQEAKDAFLGSFLYEYS
RRHPEYAVSVLLRLAKEYEATLEECCAKDDPHACYSTVFDKLKHLVDEPQNLIKQN
CDQFEKLGEYGFQNALIVRYTRKVPQVSTPTLVEVSRSLGKVGTRCCTKPESERMPC
TEDYLSLILNRLCVLHEKTPVSEKVTKCCTESLVNRRPCFSALTPDETYVPKAFDEKL
FTFHADICTLPDTEKQIKKQTALVELLKHKPKATEEQLKTVMENFVAFVDKCCAADD

KEACFAVEGPKLVVSTQTALA

In the present description, the term, "insulin precursor" may be used as one of factors replacing serum in serum free media and the like. In one embodiment, the insulin precursor may comprise the amino acid sequence of SEQ ID NO: 30 below, or consist of the amino acid sequence of SEQ ID NO: 30, but not limited thereto.

[SEQ ID NO: 30] Spacer sequence is underlined

EPKFVNQHLCGSHLVEALYLVCGERGFFYTPKAAAKGIVEQCCASVCSLYQL

ENYCN

In the present description, the term, "vector" may comprise a DNA construct comprising a base sequence of the polynucleotide operably linked to a suitable expression regulatory region (or expression regulatory sequence) so as to express a polynucleotide encoding PDI derived from *Trichoderma* sp. strain or a polynucleotide encoding a target protein in a suitable host. The expression regulatory region may comprise a promoter capable of initiating transcription, any operator sequence for regulating such transcription, a sequence encoding a suitable mRNA ribosome binding stie, and a sequence regulating termination of transcription and termination. The vector may be replicated or function independently of host genome, and may be integrated into the genome itself, after transformed into a suitable host cell.

The vector used in the present application is not particular limited, and any vector known in the art may be used. The example of the vector commonly used may include natural or recombinant plasmids, cosmids, viruses, and bacteriophages. For example, as a phage vector or cosmid vector, pWE15, M13, MBL3, MBL4, IXII, ASHII, APII, t10, t11, Charon4A, and Charon21A and the like may be used, and as a plasmid vector, pDZ-based, pBR-based, pUC-based, pBluescriptII-based, pGEM-based, pTZ-based, pCL-based and pET-based and the like may be used. Specifically, pDZ, pDC, pDCM2, pACYC177, pACYC184, pCL, pECCG117, pUC19, pBR322, pMW118, pCC1BAC, pPICZαA vectors and the like may be used.

As one example, a polynucleotide encoding PDI derived from *Trichoderma* sp. strain or a target protein may be inserted into chromosome through a vector for inserting chromosome in a cell. The insertion of the polynucleotide into chromosome may be conducted by any method known in the art, for example, homologous recombination, but not limited thereto. A selection marker to confirm whether it is inserted into chromosome may be further comprised. The selection marker is to select a cell transformed with a vector, that is, to confirm insertion of a target nucleic acid molecule, and markers that impart selectable phenotypes, such as drug resistance, auxotrophy, resistance to cytotoxic agents, or expression of surface polypeptides may be used. In an environment treated with a selective agent, only cells expressing a selection marker survive or exhibit other phenotypic traits, so transformed cells can be selected.

In the present application, the term "transformation" refers to introducing a vector comprising a specific polynucleotide into a host cell or microorganism so that a polypeptide encoded by the polynucleotide is expressed in the host cell. As long as the transformed polynucleotides may be expressed in the host cell, it may include all of these, regardless of whether they are inserted and positioned in chromosome of the host cell or positioned outside the chromosome. In addition, the polynucleotide comprises DNA and/or RNA encoding a target polypeptide. The polynucleotide may be introduced in any form, as long as it can be introduced into the host cell and expressed. For example, the polynucleotide may be introduced into the host cell in a form of an expression cassette, which is a genetic structure comprising all elements required to be expressed by itself. The expression cassette may commonly comprise a promoter operably linked to the polynucleotide, a transcription termination signal, a ribosome binding site and a translation termination signal. The expression cassette may be in a form of an expression vector capable of self-replication. In addition, the polynucleotide may be introduced into a host cell in its own form and be operably linked to a sequence required for expression in the host cell, but not limited thereto.

Furthermore, the term "operably linked" above refers to that a promoter sequence that initiates and mediates transcription of the polynucleotide encoding PDI derived from *Trichoderma* sp. strain or a target protein of the present application is functionally linked to the polynucleotide sequence.

In the present description, the term, "culture" refers to growing the mutant yeast of the present application under an approximately controlled environmental condition. The culture process of the present application may be performed according to the suitable medium and culture condition known in the art. This culture process may be easily adjusted and used by those skilled in the art depending on the selected strain. Specifically, the culture may be in a batch, continuous and/or fed-batch manner, but not limited thereto.

In the present description, the term, "medium" means a material in which nutrients required for culturing the mutant yeast of the present application are mixed as main ingredients, and supplies nutrients and growth factors and the like including water essential for survival and growth. Specifically, for the medium and other culture conditions used for culture of the mutant yeast of the present application, any medium used for culture of a common microorganism may be used without special limitation, but the mutant yeast of the present application may be cultured in a common medium containing suitable carbon sources, nitrogen sources, phosphorus sources, inorganic compounds, amino acids, and/or vitamins and the like under an aerobic condition, while adjusting the temperature, pH, and the like. Specifically, the culture medium for the yeast may be found in the document ["Yeast", Current Protocols in Molecular Biology, 2017], ["Pichia Protocols", Methods in Molecular Biology, 2007], and the like.

The carbon sources may include carbohydrates such as glucose, saccharose, lactose, fructose, sucrose, maltose, and the like; sugar alcohols such as mannitol, sorbitol, and the like, organic acids such as pyruvate, lactate, citrate, and the like; amino acids such as glutamate, methionine, lysine and the like; methanol or ethanol and the like. In addition, natural organic nutrients such as starch hydrolysates, molasses, blackstrap molasses, rice bran, cassava, sugar cane molasses, and corn steep liquor may be used, and specifically, carbohydrates such as glucose and sterilized pretreated molasses (that is, molasses converted with reducing sugar) and the like may be used, and other appropriate amounts of carbon sources may be variously used without limitation. These carbon sources may be used alone or used in combination of at least 2, but not limited thereto.

As the nitrogen sources, inorganic nitrogen sources such as ammonia, ammonium sulfate, ammonium chloride, ammonium citrate, ammonium phosphate, ammonium carbonate, ammonium nitrate and the like; organic nitrogen sources such as amino acids including glutamate, methionine, glutamine and the like, peptone, NZ-amine, meat extracts, yeast extracts, malt extracts, corn steep liquor, casein hydrolysates, fish or decomposition products thereof, defatted soybean cake or decomposition products thereof, and the like may be used. These nitrogen sources may be used alone or used in combination of at least 2, but not limited thereto.

As the phosphorus sources, potassium phosphate monobasic, potassium phosphate dibasic, or sodium-containing salts corresponding thereto and the like may be included. As the inorganic compounds, sodium chloride, calcium chloride, iron chloride, magnesium sulfate, iron sulfate, manganese sulfate, calcium carbonate and the like may be used, and other amino acids, vitamins and/or appropriate precursors and the like may be comprised. These components or precursors may be added to a medium in a batch or continuous manner. However, it is not limited thereto.

In addition, during culturing of the mutant yeast of the present application, by adding compounds such as ammonium hydroxide, potassium hydroxide, ammonia, phosphate, sulfate and the like into a medium in an appropriate method, the pH of the medium may be adjusted. Furthermore, during culturing, bubble generation may be inhibited using an antifoaming agent such as fatty acid polyglycol ester. In addition, in order to maintain the aerobic condition of the medium, oxygen or oxygen-containing gas may be injected into the medium, or in order to maintain an anaerobic and microaerobic condition, without injection of gas, or nitrogen, oxygen or carbon dioxide gas may be injected, but not limited thereto.

In the culturing step of the present invention, the culturing temperature may be maintained at 20 to 45°C, specifically, 25 to 40°C, and culturing may be performed for about 10 to 160 hours, but not limited thereto.

The recombinant protein produced by the culturing of the present invention may be secreted into the medium or be remained in cells.

The method for producing a recombinant protein of the present invention, may further comprise a step of preparing the mutant yeast of the present invention, a step of preparing a medium for culturing the mutant yeast, or a combination thereof (in any order), for example, before the culturing step.

The method for producing a recombinant protein of the present invention, may further comprise recovering a recombinant protein from the medium according to the culturing (medium on which culturing is performed) or the mutant yeast. The recovering step may be further comprised after the culturing step.

The recovering may be collecting a target protein using an appropriate method known in the art according to the culturing method of the mutant yeast of the present invention, for example, batch, continuous or fed-batch culturing method or the like. For example, centrifugation, filtration, treatment by a crystalline protein precipitating agent (salting out), extraction, sonication, ultrafiltration, dialysis, various kinds of chromatography such as molecular sieve chromatography (gel filtration), adsorption chromatography, ion exchange chromatography, affinity chromatograph and like, HPLC, or a combination of these methods may be used, and the target protein may be recovered from the medium or mutant yeast using a suitable method known in the art.

In addition, the method for producing a recombinant protein of the present invention may further comprise a purifying step. The purifying may be performed using an appropriate method known in the art. In one embodiment, when the method for producing a recombinant protein of the present application comprises both the recovering step and the purifying step, the recovering step and the purifying step may be performed discontinuously (or continuously) in any order, or may be performed at the same time or by being integrated into one step, but not limited thereto.

In addition, the present invention provides a mutant yeast for producing a target protein comprising a gene encoding PDI (protein disulfide isomerase) derived from *Trichoderma* sp. strain.

The PDI derived from *Trichoderma* sp. strain, target protein and yeast are same as described above.

The mutant yeast may be one in which a gene encoding PDI derived from *Trichoderma* sp. strain is inserted or integrated into yeast genome, and may be one in which a gene encoding PDI derived from *Trichoderma* sp. strain is present outside the yeast genome.

The mutant yeast may have increased target protein productivity as a gene encoding PDI derived from a heterologous *Trichoderma* sp. strain is introduced, compared to an unintroduced yeast.

As one example, the mutant yeast with increased target protein productivity may have the target protein productivity increased by about 1% or more, about 5% or more, about 10% or more, about 15% or more, about 20% or more, about 25% or more, about 26% or more, about 27% or more, about 28% or more, about 29% or more, about 30% or more, about 31% or more, about 32% or more, about 33% or more, about 34% or more, about 35% or more, about 36% or more, about 37% or more, about 38% or more, about 39% or more, about 40% or more, about 41% or more, about 42% or more, about 43% or more, about 44% or more, about 45% or more, about 46% or more, about 47% or more, about 48% or more, about 49% or more, about 50% or more (the upper limit is not particularly limited, for example, it may be about 200% or less, about 150% or less, about 100% or less, or about 50% or less), compared to the target protein productivity of a parent strain before mutated or a non-modified microorganism.

In another embodiment, the mutant yeast with increased productivity may have target protein productivity (or production ability, or production) increased by about 1.1 times or more, about 1.12 times or more, about 1.13 times or more, 1.15 times or more, 1.16 times or more, 1.17 times or more, 1.18 times or more, 1.19 times or more, about 1.2 times or more, 1.25 times or more, about 1.3 times or more, about 1.4 times or more, or about 1.5 times or more (the upper limit is not particularly limited, and for example, it may be about 10 times or less, about 5 times or less, about 3 times or less, or about 2 times or less), compared to a parent strain before mutated or a non-modified microorganism, but not limited thereto. The term, "about" includes a range including all ±0.5, ±0.4, ±0.3, ±0.2, ±0.1, and the like, which includes all numerical values in an equal or similar to the numerical value following the term about, but not limited thereto.

In the present description, the term, "non-modified microorganism" does not exclude a strain comprising a mutation that can occur naturally in a microorganism, and it may mean a wild-type strain or natural strain itself, or a strain before its traits are changed by genetic mutation due to natural or artificial factors. For example, the non-modified microorganism may mean a strain in which the gene encoding PDI derived from *Trichoderma* sp. strain described in the present description is not introduced or before it is introduced. The "non-modified microorganism" may be interchangeably used with "strain before modified", "microorganism before modified", "non-mutated strain", "non-modified strain", "non-mutated microorganism" or "reference microorganism". In one embodiment, the "non-modified microorganism" may be *Pichia pastoris* strain, more specifically, *Pichia pastoris* BG16 strain, but not limited thereto.

In addition, the present invention provides a composition for producing a target protein in a mutant yeast, comprising a gene encoding PDI (protein disulfide isomerase) derived from *Trichoderma* sp. strain or a recombinant vector comprising the same.

The PDI derived from *Trichoderma* sp. strain, target protein, and yeast are same as described above.

In one embodiment, the composition for producing may further comprise any appropriate excipient commonly used for a composition for producing a target protein, and such an excipient may be for example, a preservative, wetting agent, dispersant, suspending agent, buffer, stabilizer or tonicifying agent, but not limited thereto.

In addition, the present invention provides a method for increasing target protein productivity of the microorganism, or a method for imparting the target protein productivity to the microorganism, comprising introducing (for example, transforming) a polynucleotide encoding PDI (protein disulfide isomerase) derived from the *Trichoderma* sp. strain and/or a recombinant vector comprising the polynucleotide.

### [ADVANTAGEOUS EFFECTS]

The mutant yeast comprising a gene encoding PDI derived from *Trichoderma* sp. strain of the present invention highly expresses a target protein, so a target recombinant protein can be produced in high yield using it.

### [BRIEF DESCRIPTION OF THE DRAWINGS]

FIG. 1 is a diagram showing the schematic diagram of the pPICZFRT recombinant plasmid.
FIG. 2 is a diagram showing the schematic diagram of the pPICZFRT-bTRANSFERRIN(N495Q) recombinant plasmid.
FIG. 3 is a diagram showing the SDS-PAGE results for comparing the transferrin expression levels of culture supernatants of *Pichia pastoris* PDI introduced strain (CF04-1024), *Ogataea angusta* PDI introduced strain (CF04-1025) and *Trichoderma reesei* PDI introduced strain (CF04-1026).
FIG. 4 is a diagram showing the schematic diagram of the pPICZFRT-PDI(TR) recombinant plasmid.
FIG. 5 is a diagram showing the SDS-PAGE results for comparing the albumin expression levels of culture supernatants of the strain (CF04-1005) that a gene encoding bovine albumin is introduced into *Pichia pastoris* BG16 strain, and the strain (CF04-1038) that it is introduced into CF04-1035.
FIG. 6 is a diagram showing the SDS-PAGE results for comparing the insulin precursor expression levels of culture supernatants of the strain (CF04-1017) that a gene encoding a bovine insulin precursor is introduced into *Pichia pastoris* BG16 strain and the strain (CF04-1040) that it is introduced into CF04-1035.

### [MODE FOR INVENTION]

Hereinafter, the present invention will be described in more detail by examples. However, these examples are intended to illustratively describe at least one specific embodiment, and the scope of the present invention is not limited by these examples.

### Example 1. Preparation of pPICZFRT plasmid

A vector for transformation of *Pichia pastoris* was prepared by the following method.

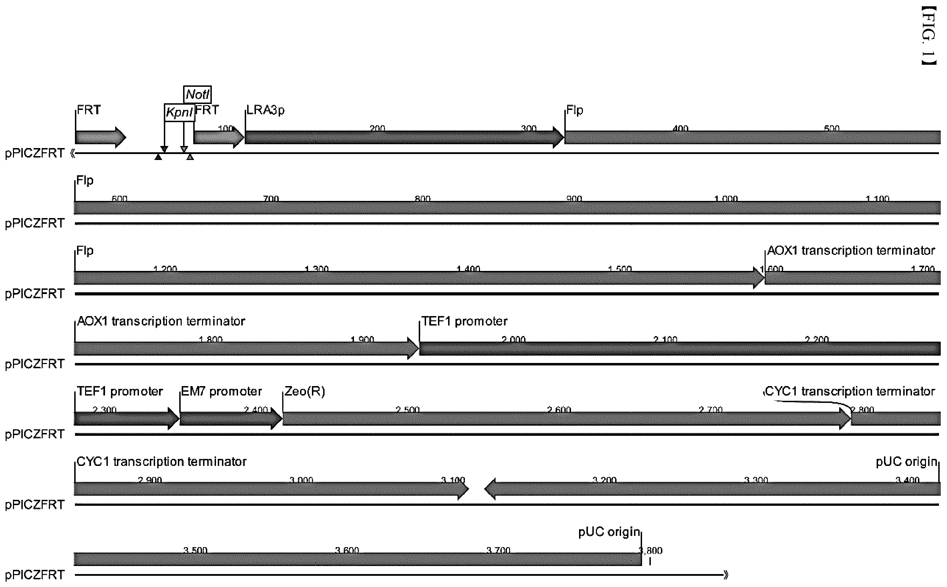

DNA (SEQ ID NO: 1) consisting of FRT site, restriction enzyme recognition site for cloning, LRA3 (L-rhamnonate dehydratase) promoter expressed under rhamnose inducing condition, and Flp recombinant enzyme was synthesized to link 1341 ~ 3593 bp regions of pPICZαA vector (Invitrogen^{™}, catalog number: V19520). The region of SEQ ID NO: 1 was amplified by PCR using DNA of SEQ ID NO: 1 synthesized for such gene manipulation as a template and using primers having base sequences of SEQ ID NO: 2 and SEQ ID NO: 3. The 1341 ~ 3593 bp regions of pPICZαA vector were amplified by PCR using the pPICZαA vector DNA as a template with the primers having base sequences of SEQ ID NO: 4 and SEQ ID NO: 5. For the PCR reaction, Pfu-X DNA Polymerase (Solg^{™}, catalog number: SPX16-R500) was used, and it followed the manufacturer's protocol. The amplified two fragments were cloned using Gibson assembly (DG Gibson et al., NATURE METHODS, VOL.6 NO.5, MAY 2009, NEBuilder HiFi DNA Assembly Master Mix) method to obtain a recombinant plasmid, and named pPICZFRT (See FIG. 1).

### Example 2. Preparation of Pichia pastoris strain expressing non-glycosylated bovine transferrin

As the amino acid sequence of bovine transferrin, 19~704th sequences other than the signal sequence in NCBI reference sequence ID: NP_803450.2 were used. In this sequence, to remove glycosylation, the 495th N (asparagine) was substituted with Q (glutamine) to use it in a non-glycosylated form, and the amino acid sequence of non-glycosylated bovine transferrin was as SEQ ID NO: 6.

In order to express a protein having the amino acid sequence of SEQ ID NO: 6 in *Pichia pastoris,* codons of DNA were optimized. The DNA codon-optimized base sequence was as SEQ ID NO: 7.

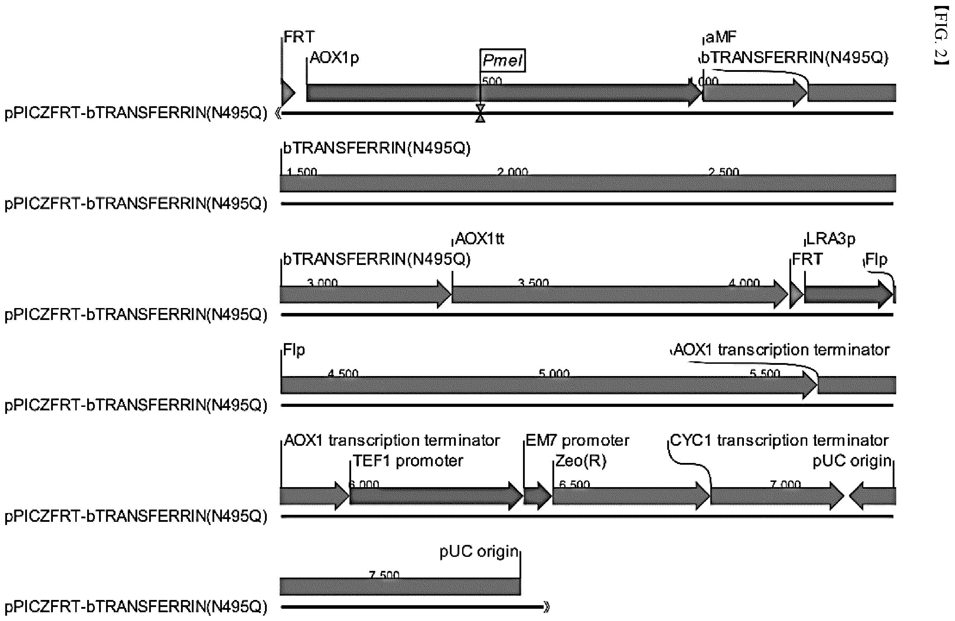

In the front of the base sequence of SEQ ID NO: 7, AOX1 promoter for methanol inducing expression, followed by an alpha mating factor sequence for secretory expression were linked, and in the back, the AOX1 transcription terminator was linked, and DNA fragments were produced by gene synthesis. The produced fragments were amplified by PCR reaction with the primers having SEQ ID NO: 8 and SEQ ID NO: 9, and using Pfu-X DNA Polymerase, and pPICZFRT was cleaved using KpnI-HF (NEB, catalog number: R3142), and NotI-HF (NEB, catalog number: R3189). The two fragments obtained in this way were cloned using Gibson assembly (DG Gibson et al., NATURE METHODS, VOL.6 NO.5, MAY 2009, NEBuilder HiFi DNA Assembly Master Mix) method to obtain a recombinant plasmid, and named pPICZFRT-bTRANSFERRIN(N495Q) (See FIG. 2).

In order to introduce the pPICZFRT-bTRANSFERRIN(N495Q) vector into the *Pichia pastoris* strain and transform it, the AOX1 promoter site was linearized using PmeI (NEB, catalog number: R0560S). After that, the linearized vector was purified using Expin^{™} PCR SV kit (GeneAll, catalog number: 103-102). As the *Pichia pastoris* strain, PPS-9016 [Pichia pastoris BG16 (pep4△, prb1△, protease deficient)] purchased from ATUM company was used. The linearized pPICZFRT-bTRANSFERRIN(N495Q) vector was introduced by electroporation according to the manual of ATUM company into *Pichia pastoris* BG16, and transformed. The transformed strain was selected in YPDS agar medium (Yeast extract 10 g/L, peptone 20 g/L, sorbitol 182.2 g/L, bacto agar 20 g/L) depending on the resistance of zeocin (250 ug/mL) antibiotic (Gibco^{™}, catalog number: R25001). Integration into the AOX1 promoter site in the genome was confirmed with a forward primer (SEQ ID NO: 10) binding to the 21 bp front of the AOX1 promoter of genome and a reverse primer (SEQ ID NO: 11) binding to the rhamnose promoter of the vector. The PCR reaction, used Phire Plant Direct PCR Kit (Thermo Scientific^{™}, catalog number: F130WH) was used, and it followed the manufacturer's protocol.

The clones whose transformation was confirmed by PCR were inoculated into 1 mL of YP (yeast extract 10 g/L, peptone 20 g/L) + 1% rhamnose liquid medium and cultured at 30°C, 200 rpm for 24 hours. In order to select clones in which the FRT region was deleted as Flp recombinase of which expression was induced by rhamnose recognized the FRT site, they were streaked in YPD agar medium (Yeast extract 10 g/L, peptone 20 g/L, bacto agar 20 g/L) and cultured at 30°C for 72 hours. Single colonies were streaked in the YPD agar medium and YPDS + zeocin agar medium, respectively, and thereby, colonies in which the FRT region was deleted and thus resistance to zeocin was lost were selected. The selected strain was named CF04-1023.

### Example 3. Preparation of Pichia pastoris strain introduced with PDI orthologue derived from various strains and confirmation of bovine transferrin expression

### Example 3-1. Preparation of vector for introducing PDI derived from various strains

In order to confirm the effect on bovine transferrin expression by introducing PDI derived from various strains into the CF04-1023 strain, the strain expressing bovine transferrin prepared in Example 2, vectors introduced with PDI derived from various strains were prepared by the following method.

As PDI orthologues, based on NCBI BlastP of the PDI amino acid sequence (SEQ ID NO: 12) of *Pichia pastoris,* PDI (SEQ ID NO: 13) of *Ogataea angusta* having homology with the amino acid sequence of SEQ ID NO: 12 of 54.63%, and PDI (SEQ ID NO: 14) of *Trichoderma reesei* having homology with it of 44.20% were selected as test candidates.

Specifically, for expression in *Pichia pastoris,* gene fragments of *Pichia pastoris* PDI were obtained by PCR with the primers having the base sequences of SEQ ID NO: 15 and SEQ ID NO: 16 from gDNA. The *Ogataea angusta* PDI and *Trichoderma reesei* PDI sequences were as SEQ ID NO: 17, SEQ ID NO: 18. Each sequence was synthesized by adding a complementary sequence to pPICZαA vector (Invitrogen^{™}, catalog number: V19020) sequence by 20bp each in the front and back for cloning. Each gene fragment was obtained by PCR using each DNA synthesized as a template with the primers having the base sequences of SEQ ID NO: 19 and SEQ ID NO: 20. Each of the obtained gene fragments was cloned using Gibson assembly (DG Gibson et al., NATURE METHODS, VOL.6 NO.5, MAY 2009, NEBuilder HiFi DNA Assembly Master Mix) method with the pPICZαA vector linearized with EcoRI-HF (NEB, catalog number: R3101S) to obtain a recombinant plasmid. The vector introduced with *Pichia pastoris* PDI was named pPICZA-PDI(PP), and the vector introduced with *Ogataea angusta* PDI was named pPICZA-PDI(OA), and the vector introduced with *Trichoderma reesei* PDI was named pPICZA-PDI(TR), respectively.

### Example 3-2. Preparation of Pichia pastoris strain introduced with PDI derived from various strains

In order to introduce the pPICZA-PDI(PP), pPICZA-PDI(OA), pPICZA-PDI(TR) vectors prepared in Example 3-1 into the CF04-1023 strain, the *Pichia pastoris* BG16 strain expressing bovine transferrin prepared in Example 2, respectively, strains introduced with PDI derived from various strains were prepared by transformation as follows.

Specifically, the AOX1 promoter site of each vector was linearized using PmeI (NEB, catalog number: R0560S). After that, each linearized vector was purified using Expin^{™} PCR SV kit (GeneAll, catalog number: 103-102). Each linearized vector was introduced into the CF04-1023 strain by electroporation according to the manual of ATUM company, and the transformed strain was selected in the YPDS agar medium depending on the resistance of zeocin (250 ug/mL) antibiotic. Integration into the AOX1 promoter site in the genome was confirmed with the forward primer (SEQ ID NO: 10) binding to the 21 bp front part and the reverse primer (SEQ ID NO: 21) binding to the TEF1 promoter of the vector. PCR reaction used Phire Plant Direct PCR Kit and followed the manufacturer's protocol. For the selected strains, the *Pichia pastoris* PDI-introduced strain was named CF04-1024, and the *Ogataea angusta* PDI-introduced strain was named CF04-1025, and the *Trichoderma reesei* PDI-introduced strain was named CF04-1026, respectively.

### Example 3-3. Confirmation of expression level of bovine transferrin by PDI derived from various strains introduced into Pichia pastoris strain

In order to confirm the expression level of bovine transferrin by PDI derived from various strains introduced into the *Pichia pastoris* strain, 4 kinds of the strains (CF04-1023, 1024, 1025, 1026) prepared in Example 3-2 were cultured to confirm expression of bovine transferrin.

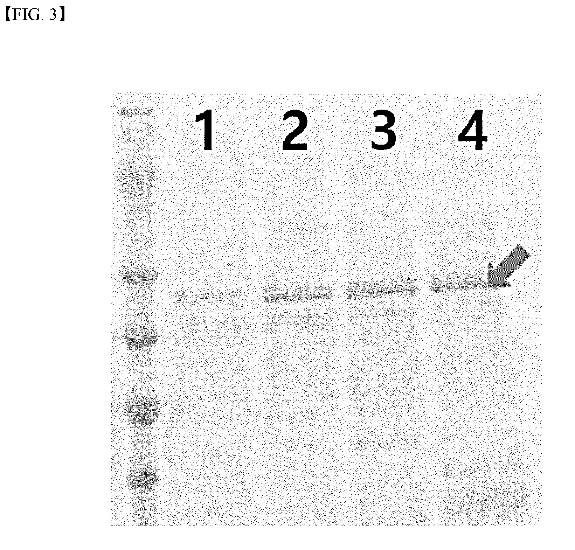

For culturing the strains, culturing was performed in 25 ml of BMMY liquid medium (1% Yeast extract, 2 % Peptone, 13.4 g/l Yeast Nitrogen Base (without amino acids), 100 mM Potassium Phosphate pH 6.0, 0.004 mg/l Biotin, 1% Methanol) using a 250 ml Baffled Erlenmeyer Flask at 30°C, 200 rpm for 96 hours, and every 24 hours, 1% methanol was additionally supplied to induce that proteins were continuously expressed under the control of the AOX1 promoter. After completing fermentation, expression was confirmed by analysis of the culture supernatants. the culture supernatants from which yeast microbial cells were sunk by centrifugation were separated using 4-20% Tris-glycine polyacrylamide gel by SDS-PAGE (Laemmli, 1970) method, and stained using DIRECTBLUE^{™} gel staining solution (SCGBIOMAX, catalog number: BDS-1000). The concentration comparison of the band corresponding to transferrin on gel was analyzed using Calibrated Densitometer (BIO-RAD, GS-900). In addition, in order to additionally confirm whether the band with the corresponding size was transferrin, it was confirmed by LC-MS/MS analysis using in gel digestion (Rosenfeld, 1992) method. The transferrin expression SDS-PAGE results were shown in FIG. 3, and the results of comparing the transferrin expression level were shown in Table 1 below.

**[Table 1]**

| No. | Strain No. | Introduced trait | Expression level (O.D) |
|---|---|---|---|
| 1 | CF04-1023 | Transferrin(N495Q) | 0 |
| 2 | CF04-1025 | Transferrin(N495Q), *Ogataea angusta* PDI | 255.49 |
| 3 | CF04-1024 | Transferrin(N495Q), *Pichia pastoris* PDI | 319.28 |
| 4 | CF04-1026 | Transferrin(N495Q), *Trichoderma reesei* PDI | 402.2 |

As a result, as shown in FIG. 3 and Table 1, it was confirmed that the strain in which PDI was not additionally introduced (CF04-1023) did not express bovine transferrin, and only in the strains in which PDI was additionally introduced, bovine transferrin was expressed. In addition, it was confirmed that the bovine transferrin expression level of the CF04-1026 strain, which is a strain in which *Trichoderma reesei* PDI was introduced, was the most excellent compared to the strains in which *Ogataea angusta* PDI and *Pichia pastoris* PDI were introduced, and the expression level was significantly higher by about 1.3 times than the strain in which *Pichia pastoris* PDI was introduced.

### Example 4. Preparation of Trichoderma reesei PDI expressing strain

Since it was confirmed that the bovine transferrin expression level of the *Pichia pastoris* strain expressing non-glycosylated bovine transferrin, in which *Trichoderma reesei* PDI was introduced was the most excellent in Example 3-3, the *Pichia pastoris* strain expressing *Trichoderma reesei* PDI was prepared by the following method.

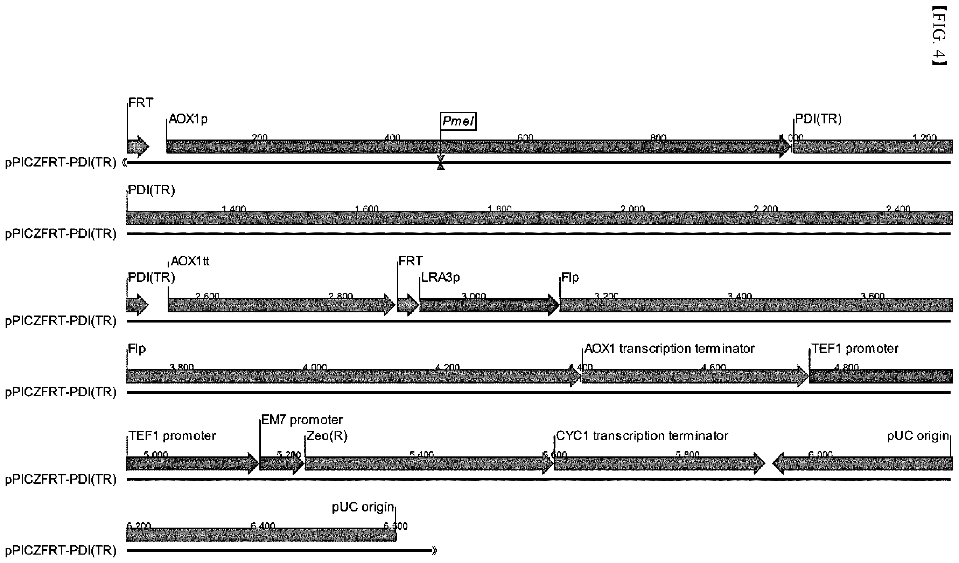

DNA fragments consisting of AOX1 *promoter-Trichoderma reesei* PDI-AOX1 terminator were obtained by PCR with Pfu-X DNA Polymerase using pPCIZA-PDI(TR) as a template with the primers having the base sequences of SEQ ID NO: 22 and SEQ ID NO: 23. pPICZFRT was cleaved using KpnI-HF, NotI-HF, and then two fragments were cloned using Gibson assembly (DG Gibson et al., NATURE METHODS, VOL.6 NO.5, MAY 2009, NEBuilder HiFi DNA Assembly Master Mix) method to obtain a recombinant plasmid, and named pPICZFRT-PDI(TR) (See FIG. 4). The pPICZFRT-PDI(TR) vector was introduced into the *Pichia pastoris* BG16 strain by the same method as the method described in Example 2, and finally, *Trichoderma reesei* PDI was integrated on the genome of the *Pichia pastoris* BG16 strain, and clones which lost resistance to zeocin were selected by excision of the FRT site. The corresponding strain was named CF04-1035 strain.

### Example 5. Confirmation of bovine albumin expression in Trichoderma reesei PDI expressing strain

In order to confirm whether the expression level of other types of proteins other than bovine transferrin was excellent, a bovine albumin gene was introduced into the *Trichoderma reesei* PDI expressing strain (CF04-1035) prepared in Example 4 and the *Pichia pastoris* BG16 strain, respectively, to compare the expression level. As the amino acid sequence of bovine albumin, the 25~607th sequences except for the signal sequence in Uniprot ID: P02769 were used, and it was as SEQ ID NO: 24.

In order to secret and express bovine albumin protein (SEQ ID NO: 24), the DNA sequence encoding it was codon-optimized, and for cloning, it was synthesized by adding a complementary sequence to pPICZαA vector (Invitrogen^{™}, catalog number: V19520) sequence by 20 bp each in the front and back, and the sequence was as SEQ ID NO: 25. A Gene fragment was obtained by PCR with Pfu-X DNA Polymerase using the synthesized DNA as a template with the primers having the base sequences of SEQ ID NO: 26 and SEQ ID NO: 27. In addition, a gene fragment to be used as a vector were obtained by PCR using the pPICZαA vector as a template with the primers having the base sequences of SEQ ID NO: 28 and SEQ ID NO: 29. The obtained two gene fragments were cloned using Gibson assembly (DG Gibson et al., NATURE METHODS, VOL.6 NO.5, MAY 2009, NEBuilder HiFi DNA Assembly Master Mix) method to obtain a recombinant plasmid. The obtained vector was named pPCIZαA-bALBUMIN. The pPCIZαA-bALBUMIN vector was introduced into the BG16 strain and CF04-1035 strain, respectively, by the same method as the method described in Example 2, and confirmation was conducted. The strain in which the gene encoding bovine albumin was introduced into the *Pichia pastoris* BG16 strain was named CF04-1005, and the strain in which it was introduced into CF04-1035 was named CF04-1038.

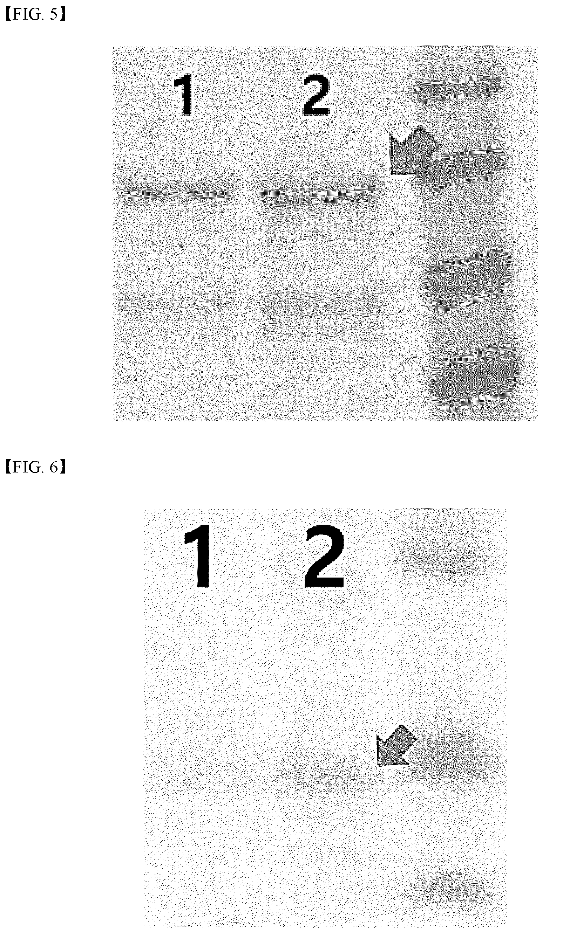

The expression level of the bovine albumin was compared by culturing the two strains, and shown in FIG. 5 and Table 2 below.

**[Table 2]**

| No. | Strain No. | Introduced trait | Expression level (O.D) |
|---|---|---|---|
| 1 | CF04-1005 | Albumin | 435.75 |
| 2 | CF04-1038 | Albumin *Trichoderma reesei* PDI | 617.33 |

As a result, as shown in FIG. 5 and Table 2, it was confirmed that the expression level of the bovine albumin of the CF04-1038 strain, a strain expressing *Trichoderma reesei* PDI was higher by about 1.4 times compared to the CF04-1005 strain, a strain in which *Trichoderma reesei* PDI was not introduced.

### Example 6. Confirmation of expression of bovine insulin precursor in Trichoderma reesei PDI expressing strain

In order to confirm whether expression of a bovine insulin precursor in the *Trichoderma reesei* PDI expressing strain is excellent, a gene encoding a bovine insulin precursor was introduced into the *Trichoderma reesei* expressing strain (CF04-1035) prepared in Example 4 and *Pichia pastoris* BG16 strain, respectively, and the expression level was compared. As the amino acid sequence of the bovine insulin precursor, 1~30th sequences corresponding to B chain and 61~81th sequences corresponding to A chain of insulin in Uniprot ID: I7CLV3 were used, and as a spacer, the EPK sequence was added to the front, and the AAK sequence was added between the B chain and A chain (Thomas Kjeldsen, 2002, JBC vol 277, issue 21). The sequence was as SEQ ID NO: 30.

In order to secret and express the bovine insulin precursor protein (SEQ ID NO: 30), the DNA sequence encoding it was codon-optimized, and for cloning, the complementary sequence to the pPICZαA vector sequence was synthesized by adding 20 bp each to the front and back, and the sequence was as SEQ ID NO: 31. A gene fragment was obtained by PCR using the synthesized DNA as a template with the primers having the base sequences of SEQ ID NO: 26 and SEQ ID NO: 27. In addition, a gene fragment to be used as a vector were obtained by PCR using the pPICZαA vector as a template with the primers having the base sequences of SEQ ID NO: 28 and SEQ ID NO: 29. The obtained two gene fragments were cloned using Gibson assembly (DG Gibson et al., NATURE METHODS, VOL.6 NO.5, MAY 2009, NEBuilder HiFi DNA Assembly Master Mix) method to obtain a recombinant plasmid. The obtained vector was named pPCIZαA-bPROINSULIN. The pPCIZαA-bPROINSULIN vector was introduced into the BG16 strain and CF04-1035 strain, respectively, by the same method as the method described in Example 2 and confirmation was conducted. The strain which the gene encoding a bovine insulin precursor was introduced into *Pichia pastoris* BG16 strain was named CF04-1017, and the strain which it was introduced into CF04-1035 was named CF04-1040.

The expression level of the bovine insulin precursor was compared by culturing the two strains. The experiment was conducted by the same method except for using 10-20% Tris-tricine polyacrylamide gel instead of 4-20% Tris-glycine polyacrylamide gel as the method described in Example 3-3, and the results were shown in FIG. 6 and Table 3 below.

**[Table 3]**

| No. | Strain No. | Introduced trait | Expression level (O.D) |
|---|---|---|---|
| 1 | CF04-1017 | Insulin Precursor | 96.2 |
| 2 | CF04-1040 | Insulin Precursor Trichoderma reesei PDI | 145.61 |

As a result, as shown in FIG. 6 and Table 3, it was confirmed that the expression level of the bovine insulin precursor of the CF04-1040 strain, a strain expressing *Trichoderma reesei* PDI was higher by about 1.5 times compared to the CF04-1017 strain, a strain in which *Trichoderma reesei* PDI was not introduced.

## Claims

1. A method for producing a recombinant protein comprising;
1) preparing a mutant yeast comprising performing the following i) and ii) steps simultaneously, sequentially, or in reverse order:
i) introducing a vector comprising a gene encoding PDI (protein disulfide isomerase) derived from *Trichoderma* sp. strain into a yeast;
ii) introducing a vector comprising a gene encoding a target protein into the yeast; and
2) culturing the mutant yeast prepared in the step 1).

2. The method for producing a recombinant protein according to claim 1, wherein the *Trichoderma* sp. strain is *Trichoderma reesei.*

3. The method for producing a recombinant protein according to claim 1, wherein the PDI (protein disulfide isomerase) derived from *Trichoderma* sp. strain of i) of step 1) above consists of the amino acid sequence of SEQ ID NO: 14.

4. The method for producing a recombinant protein according to claim 1, wherein the yeast is any one selected from the group consisting of genus *Pichia,* genus *Candida* and genus *Hansenula* strains.

5. The method for producing a recombinant protein according to claim 1, wherein the yeast is *Pichia pastoris* strain.

6. The method for producing a recombinant protein according to claim 1, wherein the target protein of ii) of step 1) above is a protein comprising at least 3 disulfide bonds.

7. The method for producing a recombinant protein according to claim 6, wherein the target protein is at least one selected from the group consisting of transferrin, albumin and insulin precursors.

8. The method for producing a recombinant protein according to claim 7, wherein the transferrin, albumin and insulin precursors are bovine-derived.

9. A mutant yeast for producing a target protein comprising a gene encoding PDI (protein disulfide isomerase) derived from *Trichoderma* sp. strain.

10. The mutant yeast according to claim 9, wherein the gene encoding PDI derived from *Trichoderma* sp. strain is inserted into yeast genome.

11. The mutant yeast according to claim 9, wherein the *Trichoderma* sp. strain is *Trichoderma reesei.*

12. The mutant yeast according to claim 9, wherein the mutant yeast is any one selected from the group consisting of genus *Pichia,* genus *Candida* and genus *Hansenula.*

13. The mutant yeast according to claim 9, wherein the mutant yeast is *Pichia pastoris* strain.

14. The mutant yeast according to claim 9, wherein the target protein comprises at least 3 disulfide bonds.

15. The mutant yeast according to claim 9, wherein the target protein is at least one selected from the group consisting of transferrin, albumin and insulin precursors.

16. The mutant yeast according to claim 15, wherein the transferrin, albumin and insulin precursors are bovine-derived.

17. A composition for producing a target protein in a mutant yeast, comprising a gene encoding PDI (protein disulfide isomerase) derived from *Trichoderma* sp. strain or a vector comprising the same.
